Europäisches Patentamt

European Patent Office

Office européen des brevets

(19)

(11) **EP 0 828 225 A1**

(12) **EUROPÄISCHE PATENTANMELDUNG**

(43) Veröffentlichungstag:
     **11.03.1998 Patentblatt 1998/11**

(51) Int. Cl.$^6$: **G06F 19/00**, A61B 5/048

(21) Anmeldenummer: **96114153.8**

(22) Anmeldetag: **04.09.1996**

(84) Benannte Vertragsstaaten:
     **AT BE CH DE DK ES FI FR GB GR IE IT LI LU MC NL PT SE**
     Benannte Erstreckungsstaaten:
     **AL LT LV SI**

(71) Anmelder:
     **SIEMENS AKTIENGESELLSCHAFT**
     **80333 München (DE)**

(72) Erfinder:
     • **Pichlmayr, Ina, Prof. Dr. med.**
       **D-30900 Wedemark (DE)**
     • **Eckert, Olaf, Dr.**
       **D-30627 Hannover (DE)**

(54)    **Verfahren und Vorrichtung zum Auswerten von EEG-Daten**

(57)    Ein Verfahren zum Auswerten von EEG-Daten für medizinische Zwecke weist die Schritte auf, EEG-Daten zu erfassen (40), Artefakte zu erkennen (50) und mittels eines neuronalen Netzerkes (64) einen Ausgangsdatenwert zu bestimmen. Bei einem Trainingsverfahren für ein neuronales Netz werden Trainingsvektoren (58) festgelegt, denen je ein Datenwert zugeordnet ist, das neuronale Netz mittels dieser Trainingsvektoren trainiert und ein Ausgangsdatenwerten für jedes Neuron bestimmt, der auf den zugeordneten Datenwerten derjenigen Trainingsvektoren basiert, die in dem durch das Neuron repräsentierten Datencluster (64) enthalten sind. Eine Verarbeitungseinrichtung und ein EEG-Monitor (36) sind dazu eingerichtet, das Auswerteverfahren für EEG-Daten durchzuführen.

Fig. 3

**START**

Ableitung von EEG-Rohdaten — 40

Aufbereitung der abgeleiteten Daten — 44    42

Digitalisierung — 46

Parametrisierung und Spektralanalyse — 48

statische Artefakterkennung — 52    50

dynamische Artefakterkennung — 54

dynamische Artefakterkennung mit autoregressiven Parametern — 56

Berechnung des gemittelten Leistungsspektrums — 60    58

Berechnung des Suppressionsparameters — 62

Ermittlung des zugeordneten neuronalen Teilnetzes — 66    64

Bestimmung des maximal aktivierten Neurons im Teilnetz — 68

Reklassifikation — 70

Anzeige des Hypnose-Index — 72

**ENDE**

**Beschreibung**

Die Erfindung betrifft ein Verfahren und eine Vorrichtung zum Auswerten von Elektroenzephalogramm-Daten (EEG-Daten) für medizinische Zwecke sowie ein Verfahren zum Training eines zu einer derartigen Auswertung geeigneten neuronalen Netzes. Unter "Auswertung" soll hierbei jedes Verfahren verstanden werden, das aus einem Strom von Eingangsdaten einen Strom von Ausgangsdaten erzeugt, die einen bestimmten, interessierenden Aspekt der Eingangsdaten widerspiegeln. Insbesondere ist die Erfindung zur Ermittlung eines Hypnose-Index aus den EEG-Daten einsetzbar. Als "Hypnose-Index" wird ein Wert bezeichnet, der die Narkosetiefe eines Patienten angibt.

Bei den heutigen Narkoseverfahren wird angestrebt, eine vollständige Anästhesie (Schmerzlosigkeit) und Hypnose (Schlafzustand) des Patienten mit einer möglichst geringen Medikamentendosis zu erreichen. Da jedoch die Wirksamkeit und Wirkungsdauer von hypnotisch wirkenden Medikamenten von Patient zu Patient unterschiedlich sind und nicht zuverlässig vorhergesagt werden können, ist vorgeschlagen worden, während und nach der Narkose die Hirnfunktion durch eine Meßeinrichtung zu erfassen.

Der Artikel "Was bringt die 3. Generation im Neuromonitoring für die klinische Praxis?" von E. Freye, K. Grabitz und W. Sandmann in der Zeitschrift "Anästhesiologie & Intensivmedizin" 3 (37), 1996, Seiten 120 - 127, enthält einen Überblick über Geräte zur Überwachung und Auswertung von EEG-Signalen zur Beurteilung der Narkosetiefe. Bei diesen Geräten wird eine Spektralanalyse der EEG-Signale durchgeführt. Die dadurch erhaltenen Parameter (Leistung in vorgegebenen Frequenzbändern, spektrale Eckfrequenz, Medianfrequenz etc.) werden wahlweise angezeigt und liefern dem Arzt Hinweise auf die Gehirnaktivität. Bei dem unter der eingetragenen Marke "Narkograph" vertriebenen Gerät erfolgt überdies eine Narkosetiefenbestimmung, die graphisch auf einem Bildschirm dargestellt wird.

Es besteht jedoch weiterhin das Problem, daß der tatsächliche Hypnosezustand eines Patienten nur schwer zu messen und aus EEG-Daten zu bestimmen ist. Wenn ein Gerät nur die bei der Spektralanalyse erhaltenen Parameter anzeigt, können diese nur von einem Spezialisten mit großem Zeitaufwand interpretiert werden. Ein angezeigter Narkosetiefenwert stimmt dagegen nicht in allen Fallen mit dem tatsächlichen Hypnosezustand des Patienten überein.

Die Erfindung hat demgemäß die Aufgabe, ein Verfahren und Gerät bereitzustellen, um aus EEG-Daten möglichst zuverlässig einen interessierenden Datenwert, beispielsweise einen Hypnose-Index, zu gewinnen.

Erfindungsgemäß wird diese Aufgabe durch ein Verfahren mit den Merkmalen des Anspruchs 1 und eine Vorrichtung mit den Merkmalen des Anspruchs 20 bzw. 21 gelöst. Kernpunkt ist dabei jeweils der Einsatz eines neuronalen Netzes zur Auswertung der EEG-Daten. Außerdem umfaßt die Erfindung ein Verfahren mit den Merkmalen des Anspruchs 14 zum Trainieren eines derartigen neuronalen Netzes.

Durch die Erfindung wird aus den EEG-Daten ein leicht erfaßbarer Ausgangsdatenwert generiert. Wenn es sich dabei um einen Hypnose-Index handelt, ist die Narkosetiefe eines Patienten objektiv und mit großer Zuverlässigkeit feststellbar. Dadurch wird eine unnötig hohe Dosierung von hypnotisch wirksamen Medikamenten vermieden, wodurch wesentliche Kosteneinsparungen im medikamentösen Bereich erzielbar sind und sich überdies die Nachbeatmungs- und Intensivbehandlungszeiten verkürzen. Ebenso wird eine Unterdosierung verhindert, bei der es vorkommen kann, daß der Patient Teile der Operation bewußt wahrnimmt. Dies ist ein für den Patienten traumatisches Erlebnis, und zwar auch dann, wenn er keine Schmerzen empfindet. Für das Erkennen solcher intraoperativer Wachzustände ist es entscheidend, daß durch den Hypnose-Index die hypnotische Komponente der Narkose erfaßt wird. Durch die Überwachung des Hypnose-Index lassen sich auch zerebrale Störungen und Komplikationen während einer Operation frühzeitig erkennen.

Neben der Narkoseüberwachung bei Operationen ist die Erfindung bei der Intensivbehandlung von Patienten einsetzbar. Eine exakte Sedierungseinstellung von Langzeitpatienten wird ermöglicht, wodurch sich die Aufwachzeiten, die Intensivbehandlungstage und die Rekonvaleszenz verkürzen. Auch hier kann die Hirnfunktion laufend kontrolliert werden, und Komplikationen des Intensivverlaufs (z.B. Sepsis, die eine zerebrale Störung bewirkt) können erkannt werden.

Ein weiteres Anwendungsgebiet der Erfindung ist die Vigilanzüberwachung von Patienten in der Psychiatrie. Auch die Wirkung von Medikamenten bei zerebralen Behandlungen (z.B. bei Arteriosklerose) kann durch die Erfindung bestimmt werden. Hierfür ist keine Langzeitüberwachung erforderlich. Es reicht vielmehr aus, den Hypnose-Index vor und nach der Verabreichung des Medikaments zu vergleichen.

Ein wichtiger Aspekt des erfindungsgemäßen Verfahrens ist die zuverlässige Erkennung von durch Artefakte verfälschten Abschnitten der EEG-Daten. Dazu ist vorzugsweise vorgesehen, Parameter der EEG-Daten mit statisch vorbestimmten Grenzwerten und/oder mit geglätteten Parameterwerten zu vergleichen. Vorzugsweise gehen nur als artefaktfrei erkannte Abschnitte der EEG-Daten in die weitere Auswertung ein. Die Abschnitte entsprechen in einem Ausführungsbeispiel Intervallen mit einer Dauer von je 2,0 s, die in der späteren detaillierten Beschreibung als "FFT-Intervalle" bezeichnet werden.

Durch das neuronale Netz werden Merkmalsvektoren klassifiziert, die eine Vielzahl von Komponenten aufweisen. Vorzugsweise gehen in den Merkmalsvektor die Signalleistung auf vorbestimmten Frequenzbändern der EEG-Daten sowie weitere Kenngrößen der EEG-Daten ein. Um die Merkmalsvektoren klassifizieren zu können, sind die Parameter

2

des neuronalen Netzes vorzugsweise durch ein Trainingsverfahren bestimmt worden, bei dem die Netztopologie nicht fest vorgegeben ist oder bei dem die Netztopologie wahrend des Trainings veränderbar ist. Damit werden besonders gute Klassifizierungsergebnisse erreicht.

Das neuronale Netz dient vorzugsweise zur Bestimmung eines Hypnose-Index; es kann jedoch die EEG-Daten auch nach anderen Kriterien auswerten und andere Indizes ermitteln.

Auch das Verfahren zum Trainieren des neuronalen Netzes wird als zur Erfindung gehörig erachtet. Mit diesem Verfahren können Parameter für Netze ermittelt werden, die eine sehr zuverlässige Clusteranalyse durchführen. Der jedem Trainingsvektor zugeordnete Ausgangsdatenwert ist bevorzugt durch lineare Interpolation zwischen fest vorgegebenen Punkten objektiv ermittelbar.

Um gute Trainingsergebnisse bei relativ geringem Rechenaufwand zu erreichen, wird das Netz vorzugsweise in getrennten Teilnetzen trainiert, die je einer Grobklasse von Merkmalsvektoren zugeordnet sind. Die Teilnetze werden vorzugsweise im Verlauf des Trainingsverfahrens aufgespalten, wenn sich keine befriedigende Clusteranalyse erzielen läßt. Damit kann das Netz schrittweise verfeinert werden. Schließlich umfaßt die Erfindung eine Verarbeitungseinrichtung bzw. einen EEG-Monitor, mit denen sich das erfindungsgemäße Analyseverfahren durchführen läßt. Vorzugsweise zeigt der EEG-Monitor den ermittelten Hypnose-Index in einer Verlaufsdarstellung an.

Unter Hinweis auf die Zeichnungen wird nun ein Ausführungsbeispiel der Erfindung beschrieben, das von den Erfindern gegenwärtig als der beste Weg erachtet wird, die Erfindung auszuführen. Es zeigen:

Fig. 1 eine schematische Darstellung des EEG-Monitors gemäß dem bevorzugten Ausführungsbeispiel während des Betriebs,

Fig. 2 eine schematische Draufsicht auf einen Kopf eines Patienten mit eingetragenen Ableitungspositionen,

Fig. 3 ein Flußdiagramm des Analyseverfahrens gemäß dem bevorzugten Ausführungsbeispiel der Erfindung,

Fig. 4 eine schematische Darstellung eines Neurons und eines Merkmalsvektors,

Fig. 5 eine die Grobklassifikation von Merkmalsvektoren darstellende Tabelle,

Fig. 6 eine Ansicht der während des Betriebs des EEG-Monitors angezeigten Bildschirmdarstellung,

Fig. 7 ein Flußdiagramm eines Trainingsverfahrens für ein neuronales Netz gemäß dem bevorzugten Ausführungsbeispiel der Erfindung,

Fig. 8 ein Flußdiagramm des bei dem in Fig. 7 gezeigten Verfahren verwendeten Lernalgorithmus, und

Fig. 9a und Fig. 9b je ein Beispiel für den zeitlichen Verlauf des Hypnose-Index in der praktischen Anwendung.

I. EEG-Monitor und Auswertungsverfahren

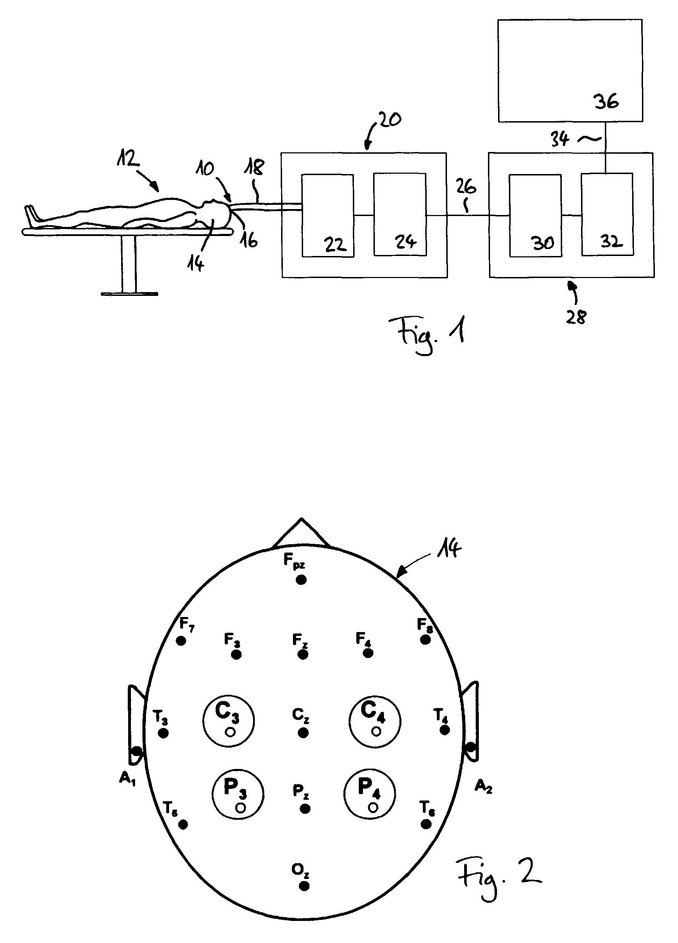

Der in Fig. 1 gezeigte EEG-Monitor zur Bestimmung eines Hypnose-Index weist eine Ableitungseinrichtung 10 zum Aufnehmen des Elektroenzephalogramms (EEG) eines Patienten 12 auf. In der gegenwärtig bevorzugten Ausführungsform wird durch die Ableitungseinrichtung 10 ein bipolares Potential zwischen zwei Positionen an dem Kopf 14 des Patienten 12 gemessen. Für diese Ein-Kanal-Ableitung sind zwei Elektroden 16, die als an sich bekannte Ag/AgCl-Sinterelektroden ausgestaltet sind, mit Klebepaste an den Kopf 14 des Patienten 12 angelegt. Als Alternative kann die Ableitungseinrichtung 10 für eine Zwei-Kanal-Ableitung eingerichtet sein. Sie weist dann vier Elektroden 16 auf. Außerdem wird bei beiden Ableitungsarten eine Erdelektrode an der Stirn des Patienten angebracht. Auch andere Elektrodenzahlen sind möglich.

Die Elektroden 16 sind über eine Leitung 18 an eine Kopfbrause 20 angeschlossen. Die Leitung 18 ist möglichst kurz, um die störempfindliche Strecke für das von den Elektroden 16 stammende unverstärkte Rohsignal, das nur einige Mikrovolt aufweist, zu verkürzen. Die Kopfbrause 20 beinhaltet eine Verstärkereinheit 22, die das Rohsignal in die Größenordnung von 1 Volt transformiert. Die Verstärkereinheit 22 ist mit einem Analogfilter 24 verbunden, der eine untere Grenzfrequenz von etwa 0,3 Hz und eine oberen Grenzfrequenz von etwa 40 Hz aufweist. Der Analogfilter 24 eliminiert insbesondere Störsignalanteile, die durch das Stromnetz induziert werden und eine Frequenz von 50 Hz aufweisen.

Über eine Leitung 26 ist der Analogfilter 24 an einen Analog/Digital-Wandler 30 angeschlossen, der in einer Verarbeitungseinrichtung 28 eingebaut ist. Die Verarbeitungseinrichtung 28 ist in dem bevorzugten Ausführungsbeispiel ein gebräuchlicher IBM-kompatibler Personalcomputer. Der Analog/Digital-Wandler 30 tastet das Eingangssignal mit 128 Hz ab und quantisiert es mit einer Auflösung von 12 Bit. Der resultierende Datenstrom wird von einer Recheneinheit 32 der Verarbeitungseinrichtung 28 in einer Reihe von Schritten weiterverarbeitet. Der in dieser Beschreibung noch im Detail dargestellte Verarbeitungsvorgang liefert Ergebnisdaten, darunter einen fortlaufenden Hypnose-Index. Die Ergebnisdaten werden von der Verarbeitungseinrichtung 28 graphisch aufbereitet und, über eine Leitung 34, an eine als Bildschirm ausgestaltete Ausgabeeinrichtung 36 geleitet und dort angezeigt.

In Fig. 2 ist schematisch das international verwendete 10/20-System von Ableitungspositionen gezeigt. Bei der gegenwärtig bevorzugten Art der Ein-Kanal-Ableitung werden die Elektroden an den Positionen $C_3$ und $P_3$ angelegt. Für die Zwei-Kanal-Ableitung werden die bipolaren Potentiale zwischen den Positionen $C_3$ und $P_3$ für einen ersten Kanal und zwischen $C_4$ und $P_4$ für einen zweiten Kanal abgeleitet. Die Position für die Erdelektrode nahe der Stirn des Patienten ist in Fig. 2 nicht gezeigt.

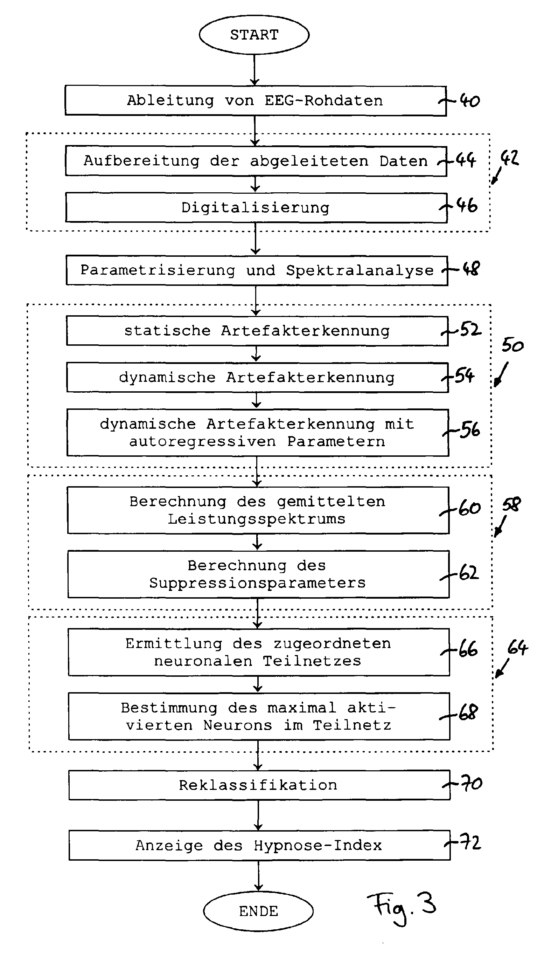

In Fig. 3 ist das erfindungsgemäße Verfahren zum Auswerten von EEG-Daten, wobei ein Hypnose-Index ermittelt wird, überblicksartig dargestellt. Grob lassen sich die folgenden Verarbeitungsstufen unterscheiden:

1. Ableiten, Aufbereiten und Digitalisieren von EEG-Daten (Schritt 40 und Block 42)
2. Parametrisierung des digitalisierten Signals und Spektralanalyse (Schritt 48),
3. Erkennung von Artefakten (Block 50),
4. Berechnung des Merkmalsvektors (Block 58),
5. Analyse durch neuronales Netzwerk (Block 64),
6. Reklassifikation (Schritt 70) und
7. Darstellung des Ergebniswertes (Schritt 72).

1. Ableiten, Aufbereiten und Digitalisieren von EEG-Daten Die von der Ableitungseinrichtung 10 aufgenommenen Rohsignale (Schritt 40) werden, wie oben unter Hinweis auf Fig. 1 beschrieben, durch Verstärkung und Filterung in der Kopfbrause 20 aufbereitet (Schritt 44) und von dem Analog/Digital-Wandler 32 digitalisiert (Schritt 46). Die digitalisierten Daten stehen der Verarbeitungseinrichtung 28 mit einer Auflösung von 12 Bit und einer Abtastfrequenz von 128 Hz zur Verfügung.

2. Parametrisierung des digitalisierten Signals und Spektralanalyse (Schritt 48)

Aus dem digitalisierten Signal berechnet die Verarbeitungseinrichtung 28 mit an sich bekannten Algorithmen eine Vielzahl von Parametern für die spätere Weiterverarbeitung.

Das Signal wird dazu zunächst unterteilt in Blöcke mit je 0,25 s Dauer (entsprechend 32 Abtastwerten), FFT-Intervalle mit je 2,0 s Dauer (entsprechend 8 Blöcken oder 256 Abtastwerten) und Bewertungsintervalle mit je 30,0 s Dauer (entsprechend 120 Blöcken oder 3840 Abtastwerten).

Für jedes Blockintervall werden der minimale und maximale Abtastwert, der Mittelwert, die Varianz (zweites Moment) und höhere Momente berechnet. Außerdem werden durch eine lineare Regressionsanalyse die Koeffizienten a und b einer den Signalverlauf während des Blockintervalls annähernden Geraden bestimmt.

Die oben genannten Parameter sowie die Maximalwerte der ersten und zweiten Ableitung des Signals werden auch für jedes FFT-Intervall bestimmt. Überdies werden die Signaldaten jedes FFT-Intervalls einer Spektralanalyse unterzogen. In dem hier beschriebenen Ausführungsbeispiel wird dazu ein an sich bekannter Algorithmus zur schnellen Fouriertransformation (FFT-Algorithmus) ausgeführt, wie er beispielsweise in dem Buch "Einführung in die Numerische Mathematik I" von Josef Stoer, 4. Auflage, Springer Verlag 1983, Seiten 71 - 81 beschrieben ist. Bevorzugt erfolgt eine Gewichtung im FFT-Intervall mittels eines Hanning-Fensters.

Durch die Spektralanalyse wird das Leistungsspektrum (Powerspektrum) des Signals im Bereich von 0 - 63,5 Hz in 128 Bändern von je 0,5 Hz bestimmt. Basierend auf diesem Leistungsspektrum wird eine Vielzahl von Parametern für jedes FFT-Intervall berechnet. Im einzelnen sind dies in dem gegenwärtig bevorzugten Ausführungsbeispiel:

- Gesamtsignalleistung,
- Medianfrequenz des Leistungsspektrums (50%-Quantil),
- spektrale Eckfrequenz des Leistungsspektrums (95%-Quantil),
- Leistung des $\delta$-Bandes (0 - 3,0 Hz),
- Leistung des $\delta_1$-Bandes (0 - 1,0 Hz),
- Leistung des $\delta_2$-Bandes (1,5 - 2,0 Hz),
- Leistung des $\delta_3$-Bandes (2,5 - 3,0 Hz),
- Leistung des $\theta$-Bandes (3,5 - 7,0 Hz),
- Leistung des $\alpha$-Bandes (7,5 - 12,0 Hz) und
- Leistung des $\beta$-Bandes (12,5 - 30,0 Hz).

Zusätzlich können weitere Parameter, insbesondere Leistungswerte bestimmter Frequenzbänder, berechnet werden.

3. Erkennung von Artefakten (Block 50)

Um eine Verfälschung des Hypnose-Index zu vermeiden, ist es entscheidend, FFT-Intervalle, in denen Artefakte aufgetreten sind, zu erkennen und auszusondern. Unter Artefakten werden temporäre Störungen im EEG-Signal verstanden, die beispielsweise durch das Ein- oder Ausschalten elektrischer Geräte (insbesondere von Hochfrequenz-Operationsgeräten), durch Bewegungen des Patienten, bei einer Intubation oder durch schlechte elektrische Kontaktgabe entstehen können.

Bei dem gegenwärtig bevorzugten Verfahren erfolgt zunächst eine statische Artefakterkennung (Schritt 52). Hierbei werden die im obigen Berechnungsschritt für jedes FFT-Intervall bestimmten Signalparameter mit fest vorbestimmten Schwell- oder Grenzwerten verglichen. Im einzelnen existieren Obergrenzen für die Signalamplitude, die Signalleistung, die erste und zweite Ableitung des Signals und für Anteile des Leistungsspektrums über 40 Hz. Überschreitet ein Parameter die ihm zugeordnete Obergrenze, wird eine Störung durch ein Artefakt angenommen. Die Signalvarianz darf dagegen eine vorgegebene Untergrenze nicht unterschreiten.

In den nächsten Verfahrensschritten wird eine dynamische Artefakterkennung für eine Vielzahl von Parametern durchgeführt (Schritte 54 und 56). Diese hat sich als ein besonders wirksames Mittel zur Analyse des EEG-Signals erwiesen.

Das Grundprinzip der dynamischen Artefakterkennung ist es, bei einem Signalparameter den jeweils aktuellen Parameterwert mit einem exponentiell geglätteten Parameterwert zu vergleichen. Übersteigt in einem FFT-Intervall der Unterschied zwischen einem aktuellen und einem geglätteten Parameterwert einen vorgegebenen Grenzwert, so wird das FFT-Intervall als artefaktbelastet markiert und zur späteren Berechnung des Merkmalsvektors (in Block 58) nicht herangezogen.

Für die dynamische Artefakterkennung in Schritt 54 können grundsätzlich alle in Schritt 48 ermittelten Parameter herangezogen werden. In dem hier beschriebenen Ausführungsbeispiel werden jedoch nur die niederfrequenten Bänder des Leistungsspektrums (Bänder $\delta_1$ bis $\delta_3$) und zusätzlich die erste und die zweite Ableitung des EEG-Signals verwendet. In einem Speicher der Verarbeitungseinrichtung 28 befindet sich für jeden dieser Parameter- und Signalwerte der bisher ermittelte geglättete Wert. Dieser Wert wird in jedem FFT-Intervall gemäß der Formel

$$\hat{P}_n = g \cdot P_n + (1 - g) \cdot \hat{P}_{n-1}$$

aktualisiert. Hierbei bedeuten $P_n$ den aktuellen Parameter- oder Signalwert im gegenwärtigen (n-ten) FFT-Intervall, $\hat{P}_n$ den für das gegenwärtige FFT-Intervall neu zu berechnenden geglätteten Wert und $\hat{P}_{n-1}$ den bisherigen geglätteten Wert, in den die FFT-Intervalle bis zum (n-1)-ten Intervall eingegangen sind. Das Maß, in dem sich der aktuelle Wert $P_n$ auf den geglätteten Wert $\hat{P}_n$ auswirkt, wird durch die fest vorgegebene Konstante g ($0 < g < 1$) bestimmt.

Als weiterer Verfahrensschritt (Schritt 56) werden auch autoregressive Parameter einer dynamischen Artefakterkennung nach dem oben mit Hinweis auf Schritt 54 beschriebenen Verfahren unterzogen. Als autoregressive Parameter bezeichnet man die Werte $a_1$, $a_2$, ..., die gemäß der Formel

$$X_n = a_1 \cdot X_{n-1} + a_2 \cdot X_{n-2} + ...$$

aus den Abtastpunkten $X_i$ (i = 1, ..., 256) eines FFT-Intervalls geschätzt werden. Die Schätzung autoregressiver Parameter $a_1$, $a_2$, ... ist in dem Buch "Zeitreihenanalyse" von Rainer Schlittgen und Bernd H.J. Streitberg, 5. Auflage, Oldenbourg Verlag 1994, auf den Seiten 121 - 132 beschrieben.

4. Berechnung des Merkmalsvektors (Block 58)

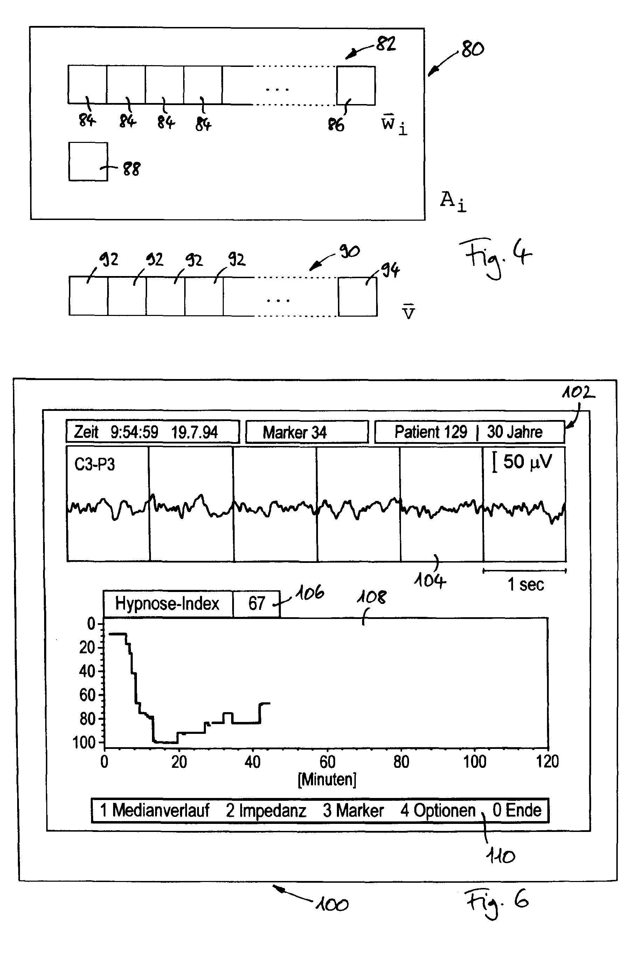

Für jedes 30-sekündige Bewertungsintervall wird ein Merkmalsvektor $\vec{v}$ berechnet, der in Fig. 4 mit dem Bezugszeichen 90 dargestellt ist. Der Merkmalsvektor 90 stellt die Eingabe für das neuronale Netz dar. Er weist in dem hier beschriebenen Ausführungsbeispiel 21 Komponenten auf, und zwar 20 Leistungskomponenten 92 und eine Suppressionsparameter-Komponente 94. Der Merkmalsvektor kann jedoch auch eine andere Anzahl von Komponenten und andere Parameter als Komponenten aufweisen. Die Leistungskomponenten 92 entsprechen den Leistungsanteilen des EEG-Signals auf 20 Frequenzbändern zwischen 0 Hz und 30,0 Hz, wobei jedes Frequenzband 1,5 Hz breit ist. Die Suppressionsparameter-Komponente 94 liefert einen Anhaltspunkt für das Auftreten eines sogenannten Burst-Suppression-Musters im EEG. Von einem Burst-Suppression-Muster spricht man dann, wenn das EEG abschnittsweise eine Nullinie aufweist, die unregelmäßig durch eine Aktivität mit einer höheren Amplitude unterbrochen ist.

Zur Bestimmung der Leistungskomponenten 92 des Merkmalsvektors 90 wird in Schritt 60 ein gemitteltes Leistungsspektrum berechnet. Dazu werden von den 15 Einzelspektren, die während des Bewertungsintervalls als Ergebnis der Spektralanalysen bestimmt wurden, zunächst diejenigen Einzelspektren verworfen, für die (d.h., für deren FFT-Intervall) in den Schritten 52 bis 56 das Auftreten eines Artefakts festgestellt worden ist. Aus den restlichen Einzelspektren wird das arithmetische Mittel im Frequenzbereich 0 - 30,0 Hz gebildet. Dabei werden je drei benachbarte Frequenzbänder von 0,5 Hz jedes Einzelspektrums zusammengefaßt, um die Leistungswerte der je 1,5 Hz breiten Frequenzbänder der Leistungskomponenten 92 zu ermitteln.

In Schritt 62 wird die Suppressionsparameter-Komponente 94 des Merkmalsvektors 90 bestimmt. Diese Komponente gibt den prozentualen Anteil von während des Bewertungsintervalls (30 s) aufgetretenen Blockintervallen (zu je 0,25 s) an, in denen die EEG-Linie sehr flach verlaufen ist.

Zur Erkennung eines "flachen" Signalverlaufs während eines Blockintervalls werden die Ergebnisse der in Schritt 48 vorgenommenen Regressionsanalyse für Blockintervalle benutzt. Ein flacher Signalverlauf wird angenommen, wenn die Steigung der den Signalverlauf während des Blockintervalls annähernden Geraden sowie der Regressionsfehler (die Streuung) vorbestimmte Obergrenzen nicht überschreiten.

## 5. Analyse durch neuronales Netzwerk (Block 64)

Der in den Schritten 60 und 62 von Block 58 erzeugte Merkmalsvektor 90 wird nun mittels eines neuronalen Netzwerks einer Clusteranalyse unterzogen. Das heißt, daß der Merkmalsvektor 90 auf seine Ähnlichkeit zu einer Anzahl N von vorbestimmten Gruppen (Clustern) von möglichen Signalvektoren untersucht wird. Jede der N Gruppen entspricht einem Neuron $A_i$ (i = 1, ..., N) des neuronalen Netzwerks. In dem gegenwärtig bevorzugten Ausführungsbeispiel sind N = 1086 Neuronen vorgesehen.

In Fig. 4 ist ein Neuron $A_i$ mit dem Bezugszeichen 80 dargestellt, das einen Synapsenvektor $\vec{w}_i$ (Bezugszeichen 82) aufweist. Der Synapsenvektor 82 weist den gleichen Aufbau wie der Merkmalsvektor 90 auf, nämlich 20 Leistungskomponenten 84 und eine Suppressionsparameter-Komponente 86. Mit anderen Worten sind alle Synapsenvektoren 82 der Neuronen 80 sowie die als Eingangswerte des neuronalen Netzes dienenden Merkmalsvektoren 90 Elemente eines Vektorraumes V. In dem Vektorraum V ist eine Norm $\|.\|$ fest vorbestimmt, und zwar in dem hier beschriebenen Ausführungsbeispiel die euklidische Norm, die definiert ist durch:

$$\| \vec{u} \| = \sqrt{\sum_{k=1}^{\dim V} (u_k)^2} \quad .$$

Hierbei bezeichnen dim V die Dimension von V (= Anzahl der Komponenten in $\vec{u}$) und $u_k$ die k-te Komponente von $\vec{u}$. Für das hier beschriebene Ausführungsbeispiel gilt dim V = 21.

Dasjenige Neuron $A_i$ (Bezugszeichen 80) des Netzes wird als maximal aktiviert erachtet, dessen Synapsenvektor $\vec{w}_i$ (Bezugszeichen 82) dem eingegebenen Merkmalsvektor $\vec{v}$ (Bezugszeichen 90) am ähnlichsten (nächsten) ist, das heißt, für das gilt:

$$\| \vec{w}_i - \vec{v} \| = \min_{j=1,\ldots,N} \| \vec{w}_j - \vec{v} \| \quad .$$

Die Bestimmung des maximal aktivierten Neurons 80 des Netzes erfolgt durch die Verarbeitungseinrichtung 28. Während es möglich wäre, für alle N Neuronen 80 nacheinander den Abstand des Merkmalsvektors 90 zu dem Synapsenvektor 82 zu bestimmen, wird in dem gegenwärtig bevorzugten Ausführungsbeispiel zunächst eine Grobklassifikation des Merkmalsvektors 90 vorgenommen (Schritt 66), durch die ein Teilnetz des neuronalen Netzes festgelegt wird.

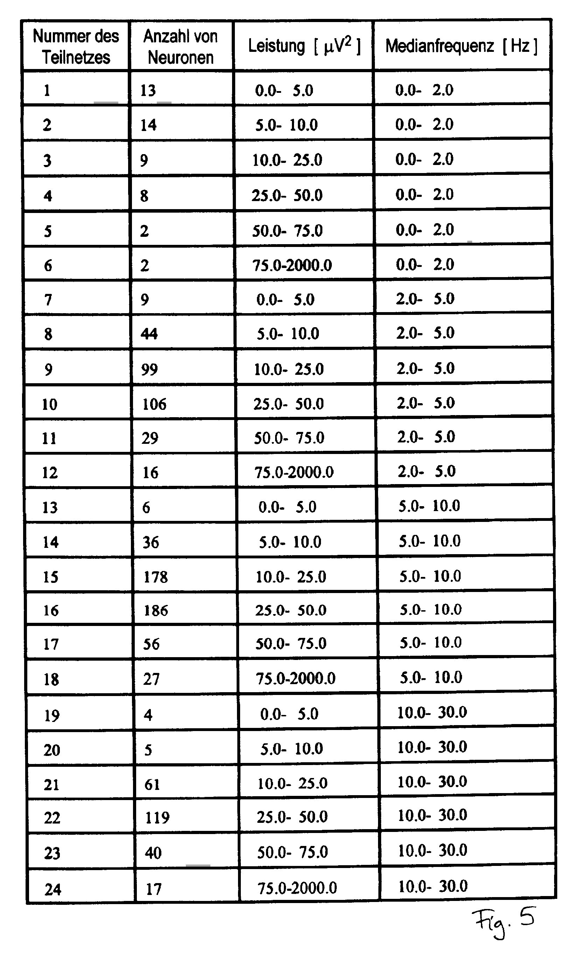

Die Grobklassifikation (Schritt 66) erfolgt gemäß der in Fig. 5 gezeigten Tabelle basierend auf der Gesamtsignalleistung und der Medianfrequenz, die sich aus den Leistungskomponenten 92 des Merkmalsvektors 90 ermitteln lassen. Ein Merkmalsvektor 90 wird einem Teilnetz zugeordnet, wenn diese Parameter in den in Fig. 5 angegebenen Intervallgrenzen enthalten sind. In dem hier beschriebenen Ausführungsbeispiel wird als Ergebnis der Grobklassifikation eines von 24 Teilnetzen bestimmt, die zwischen 2 und 186 Neuronen aufweisen.

Im darauffolgenden Schritt 68 wird der Merkmalsvektor 90 mit den Synapsenvektoren 82 der Neuronen 80 des ermittelten Teilnetzes verglichen, um gemäß der oben angegebenen Formel das maximal aktivierte Neuron 80 im Teilnetz zu bestimmen.

Die Parameter des neuronalen Netzes, also die Anzahl N der Neuronen 80 in jedem Teilnetz, die Kriterien zur Grobklassifikation und die Synapsenvektoren $\vec{w}_i$ jedes Neurons 80 sind der Verarbeitungseinrichtung 28 fest vorgegeben und verändern sich während des Betriebs nicht. Sie werden in einem Trainingsprozeß bestimmt, der nur bei der Entwicklung des Systems durchgeführt wird. Dieser Trainingsprozeß wird unten noch genau beschrieben. Es sind jedoch Ausführungsvarianten der Erfindung möglich, bei denen während des Betriebs eine langsame weitere Modifikation der Netzparameter erfolgt (adaptives Lernen).

6. Reklassifikation (Schritt 70)

Jedes Neuron 80 des Netzes weist, wie in Fig. 4 gezeigt, einen Ausgangsdatenwert 88 auf. Der Ausgangsdatenwert 88 des für einen Merkmalsvektor 90 maximal aktivierten Neurons ist der Ausgangsdatenwert des gesamten neuronalen Netzes und des erfindungsgemäßen Systems. In Ausführungsalternativen der Erfindung kann vorgesehen sein, daß der Ausgangsdatenwert 88 nicht dem Neuron 80 direkt zugeordnet ist, sondern sich erst durch weitere Berechnungen ergibt.

7. Darstellung des Ergebniswertes (Schritt 72)

Der ermittelte Ausgangsdatenwert 88, der den aktuellen Hypnose-Index angibt, wird von der Verarbeitungseinrichtung 28 graphisch aufbereitet und von der als Bildschirm ausgestalteten Ausgabeeinrichtung 36 ausgegeben.

Fig. 6 zeigt eine beispielhafte Bildschirmdarstellung 100, wie sie während einer Operation auftreten kann. Eine Kopfzeile 102 enthält allgemeine Daten wie Uhrzeit, Patientennummer und Patientenalter. In einem EEG-Fenster 104 wird das EEG-Rohsignal des Kanals $C_3$ - $P_3$ angezeigt, welches im Abstand von je 6 s erneuert wird. Eine Indexanzeige 106 gibt den augenblicklichen Wert des Hypnose-Index numerisch an. In einem Fenster 108 wird der zeitliche Verlauf des Hypnose-Index als Kurve dargestellt. Im Abstand von je 30 s wird ein neu berechneter Indexwert der Kurve hinzugefügt.

Eine Funktionsleiste 110 ermöglicht die Steuerung des EEG-Monitors und der Anzeige. Beispielsweise kann über die Option 1 eine Verlaufsdarstellung von anderen Parameterwerten (Medianfrequenz, Eckfrequenz etc.) oder eine Darstellung des gemittelten Leistungsspektrums im Fenster 108 gewählt werden. Über die Option 2 können Elektrodenimpedanzen gemessen werden. Mit der Option 3 können fortlaufend numerierte Markierungen gesetzt werden, die bei der retrospektiven Betrachtung eines Narkoseverlaufs die Zuordnung von klinisch bedeutsamen Ereignissen erleichtern.

Weitere Parameter wie Blutdruck, Herzfrequenz, Sauerstoffsättigung und Atemparameter können dem EEG-Monitor über eine Schnittstelle zugeführt werden. Diese Parameter werden zusammen mit den EEG-Daten gespeichert und können ebenfalls in der Bildschirmdarstellung 100 angezeigt werden.

II. Trainingsverfahren

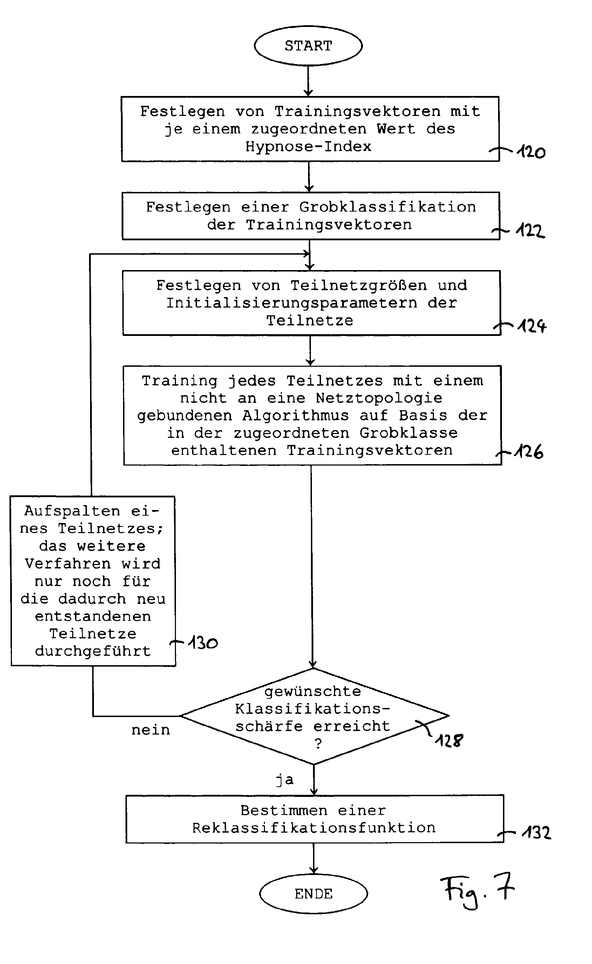

Fig. 7 zeigt in Form eines Flußdiagramms das zum Training des neuronalen Netzes durchgeführte Verfahren. Im gegenwärtig bevorzugten Ausführungsbeispiel basiert das Training auf 192 vollständigen Narkoseeinleitungen, aus denen 25549 je 30-sekündige EEG-Abschnitte ermittelt wurden. Diese EEG-Abschnitte wurden nach dem in Fig. 3 dargestellten Verfahren in Merkmalsvektoren (Trainingsvektoren) umgewandelt (Schritt 120).

Eine vollständige Narkoseeinleitung verläuft vom Wachzustand bis zum ersten Auftreten eines Burst-Suppression-Musters. Dem Wachzustand wurde der Hypnose-Index-Wert 0 zugewiesen. Da das Burst-Suppression-Muster gemäß der allgemein anerkannten Klassifikation von Kugler (H. Kugler: "Elektroenzephalographie in Klinik und Praxis", Stuttgart-New York: Thieme 1981) einen Zustand der maximalen Hypnosetiefe anzeigt, erhielt dieser Zustand den Hypnose-Index-Wert 100.

Für jeden Trainingsvektor wurde nun ein zugeordneter Wert des Hypnose-Index ermittelt, der angibt, wieviel Prozent des Zeitraums zwischen dem Infusionsbeginn (Hypnose-Index 0) bis zum ersten Auftreten des Burst-Suppression-Musters (Hypnose-Index 100) in der dem Trainingsvektor zugrundeliegenden Narkoseeinleitung bereits verstrichen waren, als der Trainingsvektor aufgenommen wurde (Schritt 120). Bei den Trainingsvektoren fällt der Narkoseindex also im zeitlichen Verlauf der Narkoseeinleitung linear ab.

Im Schritt 122 wurde eine Grobklassifikation ähnlich der in Fig. 5 gezeigten Tabelle festgelegt. Für jede Zeile der Tabelle wurden die Anzahl der Neuronen in dem zugeordneten Teilnetz und Initialisierungsparameter für die Teilnetze bestimmt (Schritt 124).

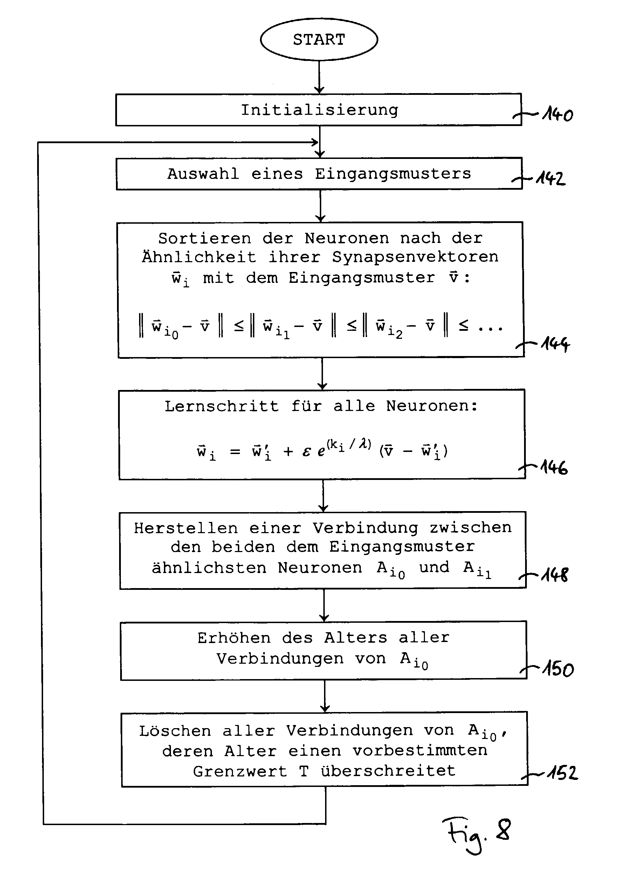

Für jedes so festgelegte Teilnetz wurde in Schritt 126 der in Fig. 8 gezeigte Trainingsalgorithmus ausgeführt, der unten genauer beschrieben wird. Nun wurde geprüft (Schritt 128), ob das gerade trainierte Teilnetz befriedigende Klassifizierungseigenschaften erreicht hatte. Kein Neuron sollte durch zwei Trainingsvektoren aktiviert werden, die stark unterschiedlichen Hypnose-Index-Werten entsprechen. Wenn ein Teilnetz solche Neuronen aufwies, also die Trainingsvektoren zu grob klassifizierte, wurde dieses Teilnetz in zwei oder mehrere neue Teilnetze aufgespalten (Schritt 130) und der Trainingsvorgang für diese Teilnetze wiederholt.

War die gewünschte Klassifikationsschärfe in allen Teilnetzen erreicht, so wurde eine Reklassifikationsfunktion bestimmt, die jedem Neuron einen Hypnose-Index als Ausgabewert zuordnet. Dieser Ausgabewert ist der arithmetische Mittelwert der Hypnose-Index-Werte derjenigen Trainingsvektoren, die das Neuron aktivieren. Da das Training so lange fortgesetzt wurde, bis jedes Neuron nur von Trainingsvektoren mit relativ ähnlichen Hypnose-Index-Werten aktiviert wurde (also von einem Cluster von Trainingsvektoren), ist der durch die Mittelwertbildung verursachte Fehler gering.

Der in dem hier beschriebenen Ausführungsbeispiel der Erfindung für das Training des neuronalen Netzes verwendete Lernalgorithmus (Schritt 126 in Fig. 7) ist in Fig. 8 genauer dargestellt. Dieser Algorithmus basiert auf dem in dem Artikel "'Neural-gas' network for vector quantization and its application to time-series prediction" von Thomas Martinetz, Stanislav Berkovich und Klaus Schulten in der Zeitschrift IEEE Transactions on Neural Networks, Band 4, Nr. 4, Juli 1993, Seiten 558 - 569 dargestellten "Neuronengas"-Algorithmus.

Grundlage des Lernalgorithmus ist ein selbstorganisierendes neuronales Netz, das auf dem Prinzip des konkurrierenden Lernens basiert. Das Netz ist nicht an eine vorgegebene Topologie gebunden, wodurch eine besonders gute Anpassung an komplexe Musterverteilungen erzielt wird. Im Gegensatz dazu gehen andere Trainingsalgorithmen von einer vorgegebenen Neuronentopologie, beispielsweise einer Anordnung der Neuronen in einem zweidimensionalen Gitter, aus und "verzerren" während des Trainings lediglich das Gitter. Auch solche Algorithmen sind jedoch für das erfindungsgemäße Verfahren einsetzbar.

Bei dem im folgenden beschriebenen Lernalgorithmus kann zwischen je zwei Neuronen $A_i$ und $A_j$ eine Verbindung bestehen. Diese nur während des Trainings benötigte Information wird durch Hilfsgrößen $C_{ij}$ für alle $i, j = 1, ..., N$ dargestellt. Ein Wert $C_{ij} = 0$ besagt, daß die Neuronen $A_i$ und $A_j$ nicht miteinander verbunden sind, während ein Wert $C_{ij} > 0$ auf eine bestehende Verbindung hinweist. Jede Verbindung weist ein durch eine natürliche Zahl dargestelltes Alter auf, das durch Hilfsgrößen $t_{ij}$ angegeben wird. Gemäß dem Algorithmus werden die folgenden Operationen ausgeführt:

(1) Initialisierung (Schritt 140):

- Ordne allen Synapsenvektoren $\vec{w}_i$ für $i = 1, ..., N$ Startwerte zu, und
- setze die Kopplungsstärken $C_{ij}$ zwischen allen Neuronen auf 0.

(2) Wähle nach dem Zufallsprinzip einen Eingangsvektor $\vec{v}$ aus den Trainingsvektoren aus (Schritt 142).

(3) Bestimme für jedes Neuron $A_i$ die Anzahl $k_i$ der Neuronen $A_j$, für die gilt:

$$\left\| \vec{w}_j - \vec{v} \right\| \; < \; \left\| \vec{w}_i - \vec{v} \right\| \; .$$

Hierfür werden die Neuronen $A_i$ nach der Ähnlichkeit ihrer Synapsenvektoren $\vec{w}_i$ mit dem Eingangsvektor $\vec{v}$ sortiert (Schritt 144):

$$\left\| \vec{w}_{i_0} - \vec{v} \right\| \; \leq \; \left\| \vec{w}_{i_1} - \vec{v} \right\| \; \leq \; \left\| \vec{w}_{i_2} - \vec{v} \right\| \; \leq \; ... \; .$$

(4) Führe einen Lernschritt für alle $i = 1, ..., N$ durch (Schritt 146):

$$\vec{w}_i \; = \; \vec{w}_i' \; + \; \varepsilon \; e^{(k_i / \lambda)} \, (\vec{v} - \vec{w}_i') \; .$$

Hierbei bezeichnet $\vec{w}_i'$ den Wert von $\vec{w}_i$ vor dem Durchführen des Lernschritts, und $\varepsilon$ und $\lambda$ sind geeignete Konstanten.

(5) Verbinde die Neuronen

$$A_{i_0} \quad \text{und} \quad A_{i_1}$$

(Schritt 148):

$$\text{Falls } C_{i_0 i_1} = 0, \text{ so setze } C_{i_0 i_1} > 0 \text{ und } t_{i_0 i_1} = 0 \, .$$

$$\text{Falls } C_{i_0 i_1} > 0, \text{ so setze } t_{i_0 i_1} = 0 \, .$$

(6) Erhöhe das Alter der Verbindungen von

$$A_{i_0}$$

für alle j = 1, ..., N (Schritt 150):

$$\text{Falls } C_{i_0 j} > 0, \text{ so setze } t_{i_0 j} = t'_{i_0 j} + 1 \, .$$

(7) Lösche diejenigen Verbindungen des Neurons

$$A_{i_0}$$

für alle j = 1, ..., N, deren Alter den Wert T überschreitet (Schritt 152):

$$\text{Falls } C_{i_0 j} > 0 \text{ und } t_{i_0 j} > T, \text{ so setze } C_{i_0 j} = 0 \, .$$

(8) Fahre bei (2) fort.

III. Experimentelle Ergebnisse

Um die Frage zu klären, mit welcher Zuverlässigkeit der EEG-Monitor gemäß dem beschriebenen Ausführungsbeispiel der Erfindung zwischen den Zuständen "Patient ist wach" und "Patient befindet sich in ausreichender Narkose" unterscheiden kann, wurden EEG-Abschnitte von 145 Patienten vor der Narkoseeinleitung und 5 Minuten nach der Intubation, also in einem Zustand voller Narkose, untersucht.

Bei den EEG-Abschnitten im Wachzustand zeigte der EEG-Monitor in 96,7 % der Fälle einen Hypnose-Index von höchstens 20 % an. Eine Messung der Medianfrequenz (Trennwert 6,0 Hz) lieferte dagegen nur in 70,8 % der Fälle das korrekte Ergebnis, und eine Messung der Eckfrequenz (Trennwert 19,5 Hz) in 68,9 % der Falle.

5 Minuten nach der Intubation zeigte der EEG-Monitor ebenfalls in 96,7 % der Fälle einen Hypnose-index von mindestens 60 % an. Bei den oben angegebenen Trennwerten führte hier die Messung der Medianfrequenz in 75,4 % der Fälle zu einem korrekten Ergebnis, und die Messung der Eckfrequenz in 69,5 % der Fälle.

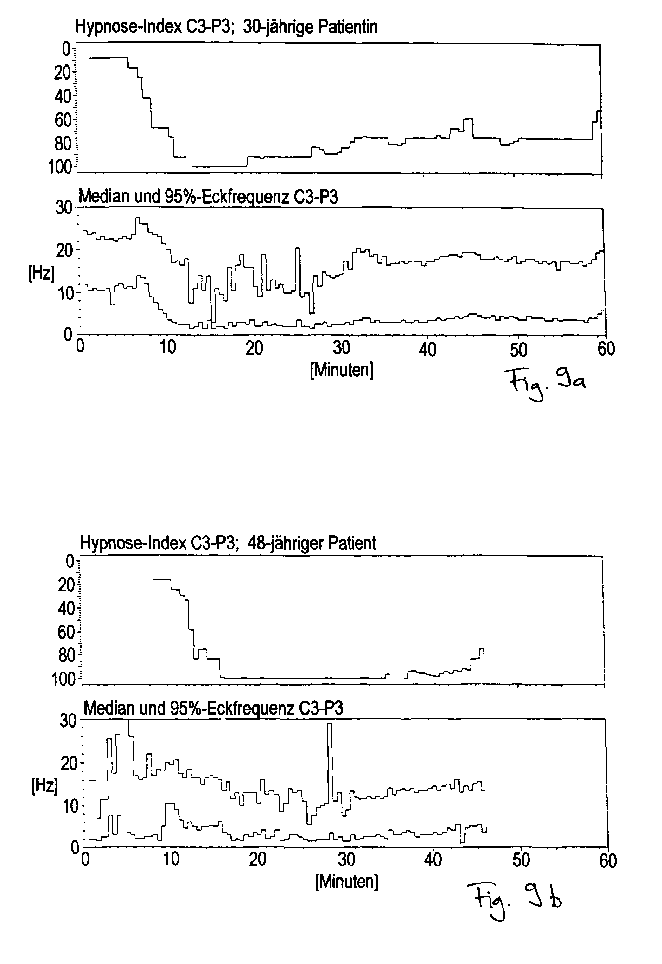

In Fig. 9a und Fig. 9b ist an zwei Beispielsfällen der zeitliche Verlauf des Hypnose-Index im Vergleich zu der Medianfrequenz und der Eckfrequenz gezeigt. In beiden Fällen zeigt der Hypnose-Index die Einleitung der Narkose durch einen ungefähr linearen Abfall und die allmähliche Narkoseausleitung an. Die Median- und Eckfrequenz reagieren dagegen in der Einleitungsphase einer Narkose nicht linear, was die Interpretation erschwert.

Bei dem in Fig. 9a gezeigten Beispiel ist insbesondere die Eckfrequenz kein stabiler Parameter. Der Hypnose-Index zeigt zum Zeitpunkt 45 Minuten eine Aufwachreaktion bei einem Hautschnitt an.

Bei dem in Fig. 9b gezeigten Beispiel reagiert die Eckfrequenz nur allmählich auf die Narkoseeinleitung.

**Patentansprüche**

1. Verfahren zum Auswerten von EEG-Daten für medizinische Zwecke, insbesondere zum Bestimmen eines Hypnose-Index, mit den Schritten:

   a) Erfassen (40) und Aufbereiten (42, 48) von EEG-Daten,
   b) Erkennen (50) von durch Artefakte verfälschten Abschnitten der EEG-Daten,
   c) Berechnen (58) eines Merkmalsvektors (90) aus den aufbereiteten EEG-Daten sowie den in Schritt b) ermittelten Artefaktinformationen,

d) Bestimmen eines Ausgangsdatenwertes (88) mittels eines neuronalen Netzes, indem der Merkmalsvektor einem durch ein Neuron (80) repräsentierten Datencluster zugeordnet wird (64) und der dem Datencluster zugeordnete Ausgangsdatenwert (88) ermittelt wird (70), und

e) Ausgeben (72) des Ausgangsdatenwertes.

2. Verfahren nach Anspruch 1,
bei dem das Aufbereiten (42, 48) der EEG-Daten in Schritt a) eine Spektralanalyse (48) umfaßt.

3. Verfahren nach Anspruch 1 oder 2, bei dem in Schritt b) Artefakte erkannt werden (50) durch

- einen Vergleich (52) von Parametern der EEG-Daten mit vorbestimmten Grenzwerten, und/oder
- einen Vergleich (54, 56) von geglätteten Parameterwerten mit aktuellen Parameterwerten, wobei ein Artefakt bei einer einen vorbestimmten Grenzwert übersteigenden Abweichung des geglätteten von dem aktuellen Parameterwert angenommen wird.

4. Verfahren nach Anspruch 3,
bei dem die zur Artefakterkennung (50) herangezogenen Parameter Leistungswerte vorbestimmter Frequenzbänder der EEG-Daten umfassen.

5. Verfahren nach einem der Ansprüche 1 bis 4,
bei dem zur Berechnung (58) des Merkmalsvektors (90) als artefaktfrei erkannte Abschnitte der EEG-Daten während eines vorbestimmten Meßintervalls herangezogen werden.

6. Verfahren nach einem der Ansprüche 1 bis 5,
bei dem der Merkmalsvektor (90) aufweist:

- Komponenten (92), die die Signalleistung in je einem vorbestimmten Frequenzband der EEG-Daten während des Meßintervalls angeben, und/oder
- mindestens eine Komponente (94), die das Auftreten eines Burst-Suppression-Musters in den EEG-Daten anzeigt.

7. Verfahren nach einem der Ansprüche 1 bis 6,
bei dem in Schritt d) zunächst eine Grobklassifikation (66) des Merkmalsvektors (90) erfolgt, durch die ein Teilnetz festgelegt wird, das dann zur Ermittlung (68) des dem Merkmalsvektor (90) zugeordneten Datenclusters dient.

8. Verfahren nach Anspruch 7,
bei dem die Grobklassifikation (66) des Merkmalsvektors (90) durch einen Vergleich von dem Merkmalsvektor (90) zugeordneten Parametern mit vorbestimmten Grenzwerten erfolgt.

9. Verfahren nach einem der Ansprüche 1 bis 8,
bei dem jedes Neuron (80) einen Synapsenvektor (82) aufweist und zur Zuordnung des Merkmalsvektors (90) zu einem der durch je ein Neuron (80) repräsentierten Datencluster dasjenige Neuron (80) ermittelt wird, dessen Synapsenvektor (82) dem Merkmalsvektor (90) am ähnlichsten ist.

10. Verfahren nach einem der Ansprüche 1 bis 9,
bei dem jedes Neuron (80) einen Parameter aufweist, der den in Schritt e) auszugebenden Ausgangsdatenwert (88) für den durch das Neuron (80) repräsentierten Datencluster angibt.

11. Verfahren nach einem der Ansprüche 1 bis 10,
bei dem die Parameter des neuronalen Netzes, insbesondere die Synapsenvektoren (82) der Neuronen (80), durch ein selbstorganisierendes Trainingsverfahren bestimmbar sind.

12. Verfahren nach Anspruch 11,
bei dem das Trainingsverfahren unabhängig von einer Topologie des neuronalen Netzes ist.

13. Verfahren nach einem der Ansprüche 1 bis 12,
bei dem der Ausgangsdatenwert (88) ein zur Narkoseführung dienender Hypnose-Index ist.

**14.** Verfahren zum Trainieren eines zum Auswerten von EEG-Daten für medizinische Zwecke geeigneten neuronalen Netzes, mit den Schritten:

a) Festlegen (120) einer Vielzahl von Trainingsvektoren, denen je ein Datenwert zugeordnet ist,
b) Festlegen (124) von Anfangsparametern des neuronalen Netzes,
c) Ausführen (126; 140 - 152) eines Trainingsalgorithmus, durch den die Parameter des neuronalen Netzes modifiziert werden, um jedem Cluster von Trainingsvektoren ein Neuron (80) zuzuordnen, und
d) Bestimmen (132) eines Ausgangsdatenwertes (88) für jedes Neuron (80), basierend auf den zugeordneten Datenwerten derjenigen Trainingsvektoren, die in dem durch das Neuron (80) repräsentierten Cluster von Trainingsvektoren enthalten sind.

**15.** Verfahren nach Anspruch 14,
bei dem in Schritt d) der Ausgangsdatenwert dem Mittelwert der zugeordneten Datenwerte derjenigen Trainingsvektoren entspricht, die in dem durch das Neuron (80) repräsentierten Datencluster enthalten sind.

**16.** Verfahren nach Anspruch 14 oder 15,
bei dem das neuronale Netze während des Trainings frei von einer vorgegebenen Topologie ist.

**17.** Verfahren nach Anspruch 16,
bei dem für alle Neuronen (80) Lernschritte (146) nach der Formel

$$\vec{w}_i \; = \; \vec{w}'_i \; + \; \varepsilon \; e^{(k_i\,/\,\lambda)} \; (\vec{v} \; - \; \vec{w}'_i)$$

durchgeführt werden, wobei $\vec{w}_i$ den Wert des Synapsenvektors (82) des jeweiligen Neurons (80) vor dem Durchführen des Lernschritts bezeichnet, $\vec{w}'_i$ den Wert von $\vec{w}_i$ nach dem Durchführen des Lernschritts, $\vec{v}$ den Trainingsvektor, $k_i$ die Anzahl der Neuronen (80), deren Synapsenvektoren (82) dem Trainingsvektor ähnlicher sind als der Synapsenvektor (82) $\vec{w}_i$, und $\varepsilon$ und $\lambda$ vorbestimmte Konstanten sind.

**18.** Verfahren nach einem der Ansprüche 14 bis 17,
bei dem zunächst eine Grobklassifikation (122) der Trainingsvektoren erfolgt und jeder so ermittelten Grobklasse von Trainingsvektoren ein Teilnetz des neuronalen Netzes zugeordnet wird (124), das nur mit den in dieser Grobklasse enthaltenen Trainingsvektoren trainiert wird (126).

**19.** Verfahren nach Anspruch 18,
bei dem eine Grobklasse von Trainingsvektoren aufgespalten wird (130), wenn sich keine befriedigenden Klassifikationsergebnisse für alle Trainingsvektoren dieser Grobklasse erhalten lassen (128).

**20.** Verarbeitungseinrichtung (28) zum Durchführen eines Verfahrens nach einem der Ansprüche 1 bis 13.

**21.** EEG-Monitor mit

-    einer Ableitungseinrichtung (10) zum Ableiten von EEG-Potentialen von einem Patienten (12),
-    einer Verstärker- und Filtereinrichtung (22, 24) zum Verstärken und Filtern der EEG-Potentiale und Erzeugen von Analogsignalen,
-    einer Verarbeitungseinrichtung (28) mit einem Analog/Digital-Wandler (30) zum Umwandeln der Analogsignale in digitale Daten, und mit einer Recheneinheit (32), um aus den digitalen Daten nach einem Verfahren nach einem der Ansprüche 1 bis 13 Ausgangsdatenwerte (88) zu ermitteln, und
-    einer Ausgabeeinrichtung (36) zur Anzeigen und/oder Ausgeben der Ausgangsdatenwerte (88).

**22.** EEG-Monitor nach Anspruch 21,
bei dem die Ausgabeeinrichtung (36) dazu eingerichtet ist, den augenblicklichen Ausgangsdatenwert (106) und/oder eine laufend aktualisierte Verlaufsdarstellung (108) der Ausgangsdatenwerte (88) anzuzeigen und/oder auszugeben.

**23.** EEG-Monitor nach Anspruch 21 oder 22,
bei dem der Ausgangsdatenwert (88) ein insbesondere zur Narkoseführung dienender Hypnose-Index ist.

Fig. 1

Fig. 2

Fig. 3

Fig. 4

$\vec{w}_i$

$A_i$

$\vec{v}$

Fig. 6

| Zeit 9:54:59 19.7.94 | Marker 34 | Patient 129 | 30 Jahre |
|---|---|---|

C3-P3                                                    $\int 50\ \mu V$

| Hypnose-Index | 67 |
|---|---|

[Minuten]

1 Medianverlauf   2 Impedanz   3 Marker   4 Optionen   0 Ende

| Nummer des Teilnetzes | Anzahl von Neuronen | Leistung [ $\mu V^2$ ] | Medianfrequenz [ Hz ] |
|---|---|---|---|
| 1 | 13 | 0.0- 5.0 | 0.0- 2.0 |
| 2 | 14 | 5.0- 10.0 | 0.0- 2.0 |
| 3 | 9 | 10.0- 25.0 | 0.0- 2.0 |
| 4 | 8 | 25.0- 50.0 | 0.0- 2.0 |
| 5 | 2 | 50.0- 75.0 | 0.0- 2.0 |
| 6 | 2 | 75.0-2000.0 | 0.0- 2.0 |
| 7 | 9 | 0.0- 5.0 | 2.0- 5.0 |
| 8 | 44 | 5.0- 10.0 | 2.0- 5.0 |
| 9 | 99 | 10.0- 25.0 | 2.0- 5.0 |
| 10 | 106 | 25.0- 50.0 | 2.0- 5.0 |
| 11 | 29 | 50.0- 75.0 | 2.0- 5.0 |
| 12 | 16 | 75.0-2000.0 | 2.0- 5.0 |
| 13 | 6 | 0.0- 5.0 | 5.0- 10.0 |
| 14 | 36 | 5.0- 10.0 | 5.0- 10.0 |
| 15 | 178 | 10.0- 25.0 | 5.0- 10.0 |
| 16 | 186 | 25.0- 50.0 | 5.0- 10.0 |
| 17 | 56 | 50.0- 75.0 | 5.0- 10.0 |
| 18 | 27 | 75.0-2000.0 | 5.0- 10.0 |
| 19 | 4 | 0.0- 5.0 | 10.0- 30.0 |
| 20 | 5 | 5.0- 10.0 | 10.0- 30.0 |
| 21 | 61 | 10.0- 25.0 | 10.0- 30.0 |
| 22 | 119 | 25.0- 50.0 | 10.0- 30.0 |
| 23 | 40 | 50.0- 75.0 | 10.0- 30.0 |
| 24 | 17 | 75.0-2000.0 | 10.0- 30.0 |

Fig. 5

START

Festlegen von Trainingsvektoren mit je einem zugeordneten Wert des Hypnose-Index ⟶ 120

Festlegen einer Grobklassifikation der Trainingsvektoren ⟶ 122

Festlegen von Teilnetzgrößen und Initialisierungsparametern der Teilnetze ⟶ 124

Training jedes Teilnetzes mit einem nicht an eine Netztopologie gebundenen Algorithmus auf Basis der in der zugeordneten Grobklasse enthaltenen Trainingsvektoren ⟶ 126

Aufspalten eines Teilnetzes; das weitere Verfahren wird nur noch für die dadurch neu entstandenen Teilnetze durchgeführt ⟶ 130

gewünschte Klassifikations-schärfe erreicht ? ⟶ 128

nein

ja

Bestimmen einer Reklassifikationsfunktion ⟶ 132

ENDE

Fig. 7

START

Initialisierung  —140

Auswahl eines Eingangsmusters  —142

Sortieren der Neuronen nach der
Ähnlichkeit ihrer Synapsenvektoren
$\vec{w}_i$ mit dem Eingangsmuster $\vec{v}$:

$$\| \vec{w}_{i_0} - \vec{v} \| \leq \| \vec{w}_{i_1} - \vec{v} \| \leq \| \vec{w}_{i_2} - \vec{v} \| \leq \ldots$$

—144

Lernschritt für alle Neuronen:

$$\vec{w}_i = \vec{w}'_i + \varepsilon \, e^{(k_i / \lambda)} (\vec{v} - \vec{w}'_i)$$

—146

Herstellen einer Verbindung zwischen
den beiden dem Eingangsmuster
ähnlichsten Neuronen $A_{i_0}$ und $A_{i_1}$  —148

Erhöhen des Alters aller
Verbindungen von $A_{i_0}$  —150

Löschen aller Verbindungen von $A_{i_0}$,
deren Alter einen vorbestimmten
Grenzwert T überschreitet  —152

Fig. 8

## Hypnose-Index C3-P3;  30-jährige Patientin

## Median und 95%-Eckfrequenz C3-P3

[Hz]

[Minuten]

Fig. 9a

## Hypnose-Index C3-P3;  48-jähriger Patient

## Median und 95%-Eckfrequenz C3-P3

[Hz]

[Minuten]

Fig. 9b

| Europäisches Patentamt | EUROPÄISCHER RECHERCHENBERICHT | Nummer der Anmeldung EP 96 11 4153 |
|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | PROCEEDINGS OF THE ANNUAL INTERNATIONAL CONFERENCE OF THE IEEE ENGINEERING IN MEDICINE AND BIOLOGY SOCIETY, Bd. 16, 3.November 1994, BALTIMORE,US, Seiten 1078-1088, XP000552483 JITENDRAN MUTHUSWAMY ET AL: "predicting depth of anesthesia using bispectral parameters in neural networks" * Zusammenfassung * | 1 | G06F19/00 A61B5/048 |
| A | * Seite 1087, linke Spalte, Zeile 1 - Seite 1088, linke Spalte, Zeile 11; Tabelle 1 *<br><br>--- | 2-4,13, 20-23 | |
| Y | WO 95 33404 A (ASPECT MEDICAL SYSTEMS, INC.) | 1 | |
| A | * Seite 6, Zeile 1 - Seite 7, Zeile 32 *<br><br>* Seite 10, Zeile 1 - Seite 14, Zeile 21 *<br>* Seite 34, Zeile 24 - Seite 37, Zeile 23; Tabellen 1-3,11 *<br><br>--- | 2-8,20, 21 | |
| A | PROCEEDINGS OF THE EIGHHTEENTH IEEE ANNUAL NORTHEAST BIOENGINEERING CONFERENCE, 12.März 1992, KINGSTON, US, Seiten 39-40, XP000410390 ASHUTOSH SHARMA ET AL: "autoregressive modeling of eeg signals for monitoring anesthetic levels" * Zusammenfassung * * Seite 39, linke Spalte, Zeile 1 - Seite 40, rechte Spalte, Zeile 8 *<br><br>---<br><br>-/-- | 1-4,14, 20,21 | **RECHERCHIERTE SACHGEBIETE (Int.Cl.6)**<br><br>G06F A61B |

Der vorliegende Recherchenbericht wurde für alle Patentansprüche erstellt

| Recherchenort | Abschlußdatum der Recherche | Prüfer |
|---|---|---|
| BERLIN | 21.Januar 1997 | Weihs, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.......................................................................
& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung

EP 96 11 4153

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | PROCEEDINGS OF THE NINTH ANNUAL CONFERENCE OF IEEE ENGINEERING IN MEDICINEAND BIOLOGY SOCIETY, Bd. 3 of 4, 13.November 1987, BOSTON, US, Seiten 1252-1253, XP000040070 C.E.THOMSEN ET AL: "monitoring of anesthetic level by eeg" * Seite 1252, linke Spalte, Zeile 1 - Seite 1253, rechte Spalte, Zeile 8; Tabellen 1,2 * | 1,7,8,14 | |
| D,A | IEEE TRANSACTIONS ON NEURAL NETWORKS , Bd. 4, Nr. 4, Juli 1993, NEW YORK, US, Seiten 558-569, XP000397414 T.M.MARTINETZ ET AL: ""neural-gas" network for vector quantization and its application to time-series prediction" * Zusammenfassung * * Seite 558, linke Spalte, Zeile 1 - Seite 560, linke Spalte, Zeile 12 * | 14-17 | |

RECHERCHIERTE SACHGEBIETE (Int.Cl.6)

Der vorliegende Recherchenbericht wurde für alle Patentansprüche erstellt

| Recherchenort | Abschlußdatum der Recherche | Prüfer |
|---|---|---|
| BERLIN | 21.Januar 1997 | Weihs, J |

EPO FORM 1503 03.82 (P04C03)